# EUROPÄISCHE PATENTSCHRIFT

(11) **EP 1 694 438 B1**
(45) Veröffentlichungstag und Bekanntmachung des Hinweises auf die Patenterteilung: **24.11.2010**
(21) Anmeldenummer: 04803522.4
(22) Anmeldetag: 04.12.2004
(51) Int. Cl.: B01J 23/72, B01J 37/00, C07D 307/08, C07D 315/00, C07C 29/136

(54) **KATALYSATOREXTRUDATE AUF BASIS KUPFEROXID UND IHRE VERWENDUNG ZUR HYDRIERUNG VON CARBONYLVERBINDUNGEN**
CATALYST EXTRUDATES BASED ON COPPER OXIDE AND THEIR USE FOR HYDROGENATING CARBONYL COMPOUNDS
PRODUIT D'EXTRUSION POUR CATALYSEUR A BASE D'OXYDE DE CUIVRE ET SON UTILISATION DANS L'HYDROGENATION DE COMPOSES CARBONYLE

(30) Priorität: 09.12.2003 DE 10357717
(43) Veröffentlichungstag der Anmeldung: 30.08.2006
(73) Patentinhaber: BASF SE, 67056 Ludwigshafen (DE)
(72) Erfinder: SCHLITTER, Stephan, 67117 Limburgerhof (DE); SCHUBERT, Olga, 67063 Ludwigshafen (DE); HESSE, Michael, 67549 Worms (DE); BORCHERS, Sabine, 76872 Erlenbach bei Kandel (DE); RÖSCH, Markus, 55276 Dienheim (DE); PINKOS, Rolf, 67098 Bad Dürkheim (DE); WECK, Alexander, 67251 Freinsheim (DE); WINDECKER, Gunther, 67067 Ludwigshafen (DE)
(86) Internationale Anmeldenummer: PCT/EP2004/013809
(87) Internationale Veröffentlichungsnummer: WO 2005/058491

(56) Entgegenhaltungen:
- EP-A- 1 228 803
- WO-A-97/34694
- DE-A1- 2 332 906
- US-A- 4 423 155
- US-A- 4 666 879

## Beschreibung

Die vorliegende Erfindung betrifft Katalysatorformkörper, bevorzugt in Form von Extrudaten, auf Basis einer Aktivmasse enthaltend Kupferoxid und ein oxidisches Trägermaterial sowie eines Binders und ihre Verwendung in Verfahren zur Hydrierung von Carbonylverbindungen.

Kupferkatalysatoren haben in der chemischen Industrie einen weiten Anwendungsbereich. Insbesondere zur Hydrierung von Carbonylverbindungen wie beispielsweise Carbonsäureestern und -anhydriden, von Aldehyden oder Nitroverbindungen sind geträgerte Kupferkatalysatoren eine geeignete Wahl. Üblicherweise werden diese Katalysatoren in Form von Tabletten als Formkörper produziert und eingesetzt.

Eine Möglichkeit zur Verbesserung dieser Katalysatoren, beispielsweise hinsichtlich ihrer Selektivität bei Folgereaktionen, besteht darin, ihre Formkörpergeometrie und Porosität für die Anwendung zu optimieren. Dieser Optimierung von Katalysatortabletten sind jedoch Grenzen gesetzt, weil kleine, poröse Tabletten entweder keine ausreichende mechanische Stabilität mehr aufweisen oder in der Herstellung überproportional kostenaufwändig werden.

Der Erfindung lag daher die Aufgabe zu Grunde, ein optimiertes Katalysatorsystem zu finden, welches die oben genannten Nachteile der Katalysatortabletten gemäß dem Stand der Technik behebt. Überraschenderweise wurde gefunden, dass mittels geeigneter Extrusionsverfahren und geeigneter Binder und Bindermengen auf preiswertere Art und Weise Katalysatorformkörper mit den gewünschten Eigenschaften hergestellt werden können.

Katalysatorformkörper auf Basis von extrudierten kupferhaltigen Materialien sind prinzipiell in der Literatur bekannt.

So beschreiben Müller et al. im Journal of Catalysis, 218, 2003, S. 419-426 die Herstellung von Kupferoxid/Zinkoxid-Katalysatorextrudaten mit Aluminiumoxidhydrat als Bindermaterial, welches im fertigen Katalysator als gamma-Aluminiumoxid vorliegt. Zinkfreie Katalysatoren, wie sie für die hier beschriebenen Anwendungen vorteilhaft sind, werden dort nicht beschrieben. Nachteilig an diesen Extrudaten ist ihre deutlich vermindert, aktive Oberfläche im Vergleich zum nicht extrudierten Vergleichssystem. Zudem wird beim beschriebenen Typ von Extrudat die Aktivmasse des Katalysators (CuO/ZnO) durch den Binder (Al₂O₃) verdünnt, was sich zusätzlich aktivitätsmindemd auswirken kann.

In der WO 97/34694 wird in Beispiel 22 die Herstellung von Katalysatorextrudaten aus einem cogefällten CuO/Al₂O₃ Pulver ohne jeden weiteren Zusatz beschrieben. Die dabei erzielte mechanische Stabilität des Formkörpers sowie die Porosität sind jedoch nur teilweise befriedigend. Die in den Ausführungsbeispielen erzielte Porosität entspricht lediglich derjenigen von tablettiertem Material.

Es besteht daher die Aufgabe einen Katalysator zur Verfügung zu stellen, der einfach herzustellen ist, hohe mechanische Stabilität und eine gute katalytische Aktivität insbesondere für die Hydrierung von Carbonylverbindungen aufweist.

Es wurde nun gefunden, dass ein Katalysatorformkörper von makroskopisch einheitlichem Strukturaufbau, der als Aktivkomponenten Kupferoxid und ein oxidisches Trägermaterial und zudem einen dem Trägermaterial angepassten oxidischen Binder aufweist, bevorzugt in Form eines Extrudates technisch leicht herstellbar ist als auch zu hohen Aktivitäten und Selektivitäten sowie zu einer hohen Stabilität führt.

Gegenstand ist ein Katalysatorformkörper von makroskopisch einheitlichem Strukturaufbau, bevorzugt in Form eines Extrudates, enthaltend 5 bis 85 Gew.-% Kupferoxid und ein oxidisches Trägermaterial, der in der Aktivmasse und als Binder das gleiche oxidische Trägermaterial aufweist, **dadurch gekennzeichnet, dass**
a) der Formkörper ein Porenvolumen > 0,15 ml/g im Bereich der Porendurchmesser 10 nm bis 100 nm zeigt und
b) das oxidische Trägermaterial im Formkörper sowohl feindispers verteilt vorhanden ist, als auch zu einem Volumenanteil von 1 bis 95 Vol.-% des Formkörpers in partikulärer Form, vorliegt.

Der erfindungsgemäße Katalysator weist als Aktivkomponente Kupferoxid, ggf. in reduzierter Form, und ein oxidisches Trägermaterial aus der Gruppe Aluminiumoxid, Titanoxid, Zirkonoxid, Siliciumdioxid, den Manganoxiden bzw. Mischungen davon auf. Bevorzugt wird als Trägermaterial Aluminiumoxid verwendet, welches insbesondere als röntgenamorphes Oxid im Katalysator vorliegt. Der Katalysator kann optional eines oder mehrere weitere Metalle bzw. deren Verbindungen, vorzugsweise Oxide, aus den Gruppen 1 bis 14 (IA bis VIIIA und IB bis IVB der alten IUPAC-Nomenclatur) des Periodensystems der Elemente in Anteilen bis zu 20 Gew.-%, bevorzugt bis zu 10 Gew.-% enthalten. Falls der Katalysator Zinkoxid als optionales weiteres Metall enthält, liegt der Anteil an Zinkoxid bevorzugt bei < 5 Gew.-%, besonders bevorzugt < 1 Gew.-% und insbesondere < 500 ppm.

Anstelle der Oxide selbst kann die Aktivkomponente auch ganz oder teilweise geeignete Precursorverbindungen des Kupferoxids und des oxidischen Trägermaterials enthalten, beispielsweise in Form von Oxidhydraten, Hydroxiden und/oder Carbonaten. Das Kupferoxid liegt in der Aktivkomponente bevorzugt innig vermischt mit dem oxidischen Trägermaterial vor.

Der Anteil an Kupferoxid bzw. der Precursorverbindungen des Kupferoxids in der Aktivkomponente liegt bei mehr als 10 bis 98 Gew.-%, bevorzugt 30 bis 95 Gew.-%, besonders bevorzugt bei 50 bis 95 Gew.-% und insbesondere bei 80 bis 90 Gew.-% (be rechnet im geglühten Zustand, d.h. bezogen auf 100 % als Metalloxid vorliegende Aktivkomponente).

Der erfindungsgemäße Katalysator weist zudem mindestens einen Binder auf. Der Binder enthält das gleiche oxidische Trägermaterial, welches auch in der Aktivkomponente vorhanden ist, oder bevorzugt einen Precursor dieses Trägermaterials. Enthält die Aktivkomponente neben Kupferoxid beispielsweise hauptsächlich Aluminiumoxid oder Precursoren davon, so enthält der Binder ebenfalls Aluminiumoxids und/oder Precursoren davon, insbesondere Aluminiumoxidhydrate, besonders bevorzugt Böhmit oder pseudo-Böhmit. Enthält die Aktivkomponente neben Kupferoxid beispielsweise hauptsächlich Siliciumoxid oder Precursoren davon, so enthält der Binder ebenfalls Siliciumoxid und/oder Precursoren davon, insbesondere Kieselsäuren, Kieselsäureester oder Ester alkylierter Kieselsäuren. Die Ausführungsform des erfindungsgemäßen Katalysator, bei der Aluminiumoxid und/oder Aluminiumoxid-Precursoren als Bestandteil sowohl in der Aktivkomponente als auch im Binder enthalten sind, ist bevorzugt.

Der Anteil des Kupferoxids an der Gesamtmasse des Katalysators liegt bei 5 bis 85 Gew.-%, vorzugsweise bei 10 bis 70 Gew.-%., besonders bevorzugt bei 40 bis 65 Gew.-%.

Der erfindungsgemäße Katalysator liegt als Extrudat vor. Dem Extrusionsansatz werden typischerweise außer den vorgenannten noch weitere Komponenten und Hilfsmittel zugesetzt. Regelmäßig werden Wasser und ggf. Säuren bzw. Basen eingesetzt. Dazu als Hilfsstoffe organische und anorganische Stoffe, die zu einer verbesserten Verarbeitung während der Katalysatorextrusion und/oder zu einer weiteren Erhöhung der mechanischen Festigkeit und/oder zur gewünschten Porosität der Katalysatorformkörper beitragen. Derartige Hilfsmittel sind dem Fachmann bekannt; Beispiele umfassen Graphit, Stearinsäure, Kieselgel, Siloxane, Zelluloseverbindungen, Stärke, Polyolefine, Kohlenhydrate (Zucker), Wachse oder Alginate.

Die optional im Katalysator enthaltenen, weiteren Metalle bzw. deren Verbindungen, vorzugsweise Oxide, aus den Gruppen 1 bis 14 (IA bis VIIIA und IB bis IVB der alten IUPAC-Nomenclatur) des Periodensystems, können in der Aktivmasse und/oder im Binder enthalten sein und/oder dem Extrusionsansatz als weitere Komponente zugegeben werden.

Bei der bevorzugten Verwendung von Böhmit als Binder werden bevorzugt wässrige Säuren, insbesondere Ameisensäure oder Salpetersäure, dem Extrusionsansatz zugemischt, neben ggf. Carboxymethylzellulose, Kartoffelstärke oder Stearinsäure.

Von dem im Katalysatorextrudat enthaltenen oxidischen Trägermaterial, d.h. den Materialien aus der Gruppe Aluminiumoxid, Titanoxid, Zirkonoxid, Siliciumdioxid, den Manganoxiden bzw. Mischungen davon, stammen 10 bis 98 Gew.-% aus dem eingesetzten Binder, bevorzugt 15 bis 95% und insbesondere 20 bis 50 Gew.-%. Entsprechend entstammen nur 2 bis 90 Gew.-%, bevorzugt 5 bis 85 Gew.-% und besonders bevorzugt nur 50 bis 80 Gew.-% des im Extrudat enthaltenen Trägermaterials aus der Aktivkomponente (jeweils berechnet als oxidisches Trägermaterial).

Die bei der Extrusion einzusetzenden Mengenverhältnisse aus Aktivmasse und Binder ergeben sich aus der Zusammensetzung von Aktivmasse und Binder, der Zielzusammensetzung des Katalysators bzw. dem angestrebten Anteil an Trägermaterial, welches aus dem Binder resultieren soll.

Durch die Wahl einer Aktivkomponente mit bevorzugt hohem Anteil an Kupferoxid und dem Einsatz einer Mindestmenge an Binder, welcher das gleiche Trägermaterial wie die Aktivkomponente enthält, können gleichzeitig sehr aktive und mechanisch ausgesprochen stabile Extrudate enthalten werden. Mit der erfindungsgemäßen Herstellung wird sowohl die nachteilige Verdünnung des Katalysators durch ein in der Reaktion inerten Binderanteil vermieden, als auch durch den geeigneten Binderanteil eine hohe Festigkeit erzielt.

Die Aktivmassen lassen sich nach dem Fachmann bekannten Methoden herstellen. Bevorzugt sind Verfahren, bei denen das Kupferoxid fein verteilt und innig vermischt mit den anderen Bestandteilen der Aktivmasse und dem sauren Oxid anfällt. Besonders bevorzugt werden die entsprechenden Metallsalze und/oder Hydroxide aus wässriger Lösung ausgefällt, gewaschen, getrocknet und calciniert. Als Metallsalze kommen beispielsweise Nitrate, Sulfate, Carbonate, Chloride, Acetate oder Oxalate in Betracht. Anschließend wird dieses Ausgangsmaterial nach bekannten Methoden zu den Formkörpern durch Extrudieren, gegebenenfalls unter Zusatz von Hilfsmitteln verarbeitet werden.

Die Extrudate werden durch beispielsweise Kneten oder Kollern der Ausgangsverbindungen mit dem Binder, beispielsweise Böhmit oder p-Böhmit (AIOOH) erhalten und anschließend calciniert. Der Binder kann vor dem Extrudieren vorbehandelt werden. Bevorzugt geschieht dies mit Säure, wie zum Beispiel mit Ameisensäure oder Salpetersäure . Andere Hilfsmittel wie zum Beispiel Porenbildner wie Carboxymethylzellutose, Kartoffelstärke oder Stearinsäure können zusätzlich vor oder während dem Extrudieren zugegeben werden.

Die erfindungsgemäßen Katalysatoren lassen sich in verschiedenen Extrudatformen herstellen. Beispielhaft genannt seien zylindrische Extrudate, Stern- oder Rippstränge, Triloben, Hohlstränge und Wabenstränge. Die typischen Durchmesser dieser Extrudate liegen bei 0,5 bis 10 mm, bevorzugt 1 bis 6 mm, besonders bevorzugt 1,5 bis 3 mm. Das mittlere Verhältnis von Länge zu Durchmesser liegt bei 0,2:1 bis 20:1, bevorzugt 0,7:1 bis 10:1, besonders bevorzugt 1:1 bis 5:1.

Im Anschluss an die Formgebung werden die Katalysatoren getrocknet und ggf. calciniert. Im Fall der bevorzugten Katalysatoren auf Basis von Kupferoxid und Aluminiumoxid werden bevorzugt Calcinierbedingungen gewählt, bei der das Aluminiumoxid überwiegend röntgenamorph und das Kupferoxid als feinkristalliner Tenorit vorliegt. Bevorzugt lassen sich nur <30 Gew.-%, besonders bevorzugt <20 Gew.-% und insbesondere <10 Gew.-% des Aluminiumoxids als kristalline Phase per Pulverdiffraktorgramm detektieren.Übliche Calciniertemperaturen für diese bevorzugten Katalysatoren liegen bei 300 bis 800°C, bevorzugt 500 bis 700°C und besonders bevorzugt bei 550 bis 650°C bei Calcinierzeiten von 5 min bis 5 h, bevorzugt 10 min bis 2 h.

Die BET-Oberfläche der Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) des reduzierten Katalysators beträgt im Einbauzustand > 1 m²/g, vorzugsweise > 3 m²/g, insbesondere > 6 m²/g Kupfer.

Der erfindungsgemäße Katalysator weist einen makroskopisch einheitlichen Strukturaufbau auf. Seine Komponenten, d.h. das Kupferoxid, das oxidische Trägermaterial und der Binder, liegen miteinander vermischt vor, wodurch der Katalysator keine großen Bereiche (im mm-Bereich) mit unterschiedlich aufgebauten Bestandteilen aufweist, wie dies beispielsweise bei Schalenkatalysatoren der Fall ist.

Auf der mikroskopischen Ebene hingegen liegt das Trägermaterial sowohl feindispers verteilt in der Aktivmasse vor, als auch in partikulärer Form resultierend aus dem zugesetzen Binder. Gemäß dieser bevorzugten Ausführungsform bestehen 1 bis 95 %, bevorzugt 3 bis 80 % und insbesondere 5 bis 50 % des Volumens des Katalysatorformkörpers aus Trägerpartikeln, d.h. aus Partikeln mit einem Durchmesser größer als ca. 2 µm bzw. einem Volumen größer als ca. 4 µm³.

Gemäß der Erfindung werden Katalysatorformkörper verwendet, die eine definierte Porosität im Bereich der großen Mesoporen bzw. kleiner Makroporen aufweisen. Diese Katalysatoren zeigen ein Porenvolumen von > 0,15 ml/g, bevorzugt > 0,20 ml/g, besonders bevorzugt > 0,30 ml/g für Porendurchmesser im Bereich 10 nm bis 100 nm.

Die erwähnten Porositäten wurden durch Quecksilber-Intrusion nach DIN 66133 bestimmt. Es wurden die Daten im Porendurchmesserbereich von 4 nm bis 300 nm ausgewertet. Die Einstellung der erfindungsgemäßen Porosität kann nach dem Fachmann bekannten Methoden erfolgen, beispielsweise durch Wahl der Teilchengrößenverteilung von Aktivkomponente und insbesondere derjenigen des Binders, durch die Parameter des Formgebungsverfahrens und/oder durch Art und Menge der eingesetzten Zuschlags- und Hilfsstoffe.

In der Anwendung als Hydrierkatalysator für Carbonylverbindungen wird der Katalysator in reduzierter, aktivierter Form eingesetzt. Die Aktivierung erfolgt mit reduzierenden Gasen, vorzugsweise Wasserstoff oder Wasserstoff/Inertgas-Gemischen, entweder vor oder nach dem Einbau in die Reaktoren, in welchen das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 350°C. Bei der sogenannten in-situ-Aktivierung erfolgt die Aktivierung beim Hochfahren der Anlage durch Kontakt mit Wasserstoff bei erhöhter Temperatur.

Der erfindungsgemäße Katalysator eignet sich zur Hydrierung von Carbonylverbindungen wie z.B. Aldehyden und Ketonen zu den entsprechenden Alkoholen, wobei aliphatische und cycloaliphatische gesättigte und ungesättigte Carbonylverbindungen bevorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkoholen hydriert. Der Begriff "Carbonylverbindungen", wie er im Rahmen der Erfindung verwendet wird, umfasst alle Verbindungen, die eine C=O-Gruppe aufweisen, einschließlich Carbonsäuren und deren Derivaten. Selbstverständlich können auch Gemische aus zwei oder mehr als zwei Carbonylverbindungen gemeinsam hydriert werden. Ferner kann auch die einzelne, zu hydrierende Carbonylverbindung mehr als eine Carbonylgruppe enthalten.

Bevorzugt wird der erfindungsgemäße Katalysator zur Hydrierung aliphatischer Aldehyde, Hydroxyaldehyde, Ketone, Säuren, Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind. Ferner können auch oligomere Verbindungen, die auch mehr als 30 Carbonylgruppen enthalten, hydriert werden.

Als Beispiel für aliphatische Aldehyde sind zu nennen:
Formaldehyd, Propionaldehyd, n-Butyraldehyd, iso-Butyraldehyd, Valeraldehyd, 2-Methylbutyraldehyd, 3-Methylbutyraldehyd (isovaleraldehyd), 2,2Dimethylpropionaldehyd (Pivalinaldehyd), Capronaldehyd, 2-Methylvaleraldehyd, 3Methylvaleraldehyd, 4-Methylvaleraldehyd, 2-Ethylbutyraldehyd, 2,2Dimethylbutyraldehyd, 3,3-Dimethylbutyraldehyd, Caprylaldehyd, Caprinaldehyd, Glutardialdehyd.

Neben den genannten kurzkettigen Aldehyden sind insbesondere auch langkettige aliphatische Aldehyde geeignet, wie sie beispielsweise durch Oxosynthese aus linearen a-Olefinen erhalten werden können.

Besonders bevorzugt sind Enalisierungsprodukte, wie z.B. 2-Ethylhexenal, 2Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydroxy-2-ethylhexanal (Butylaldol), 3-Hydroxy-2methylpentanal (Propienaldol), 2-Methylolpropanal, 2,2-Dimethylolpropanal, 3-Hydroxy-2-methylbutanal, 3-Hydroxypentanal, 2-Methylolbutanal, 2,2-Dimethylolbutanal, Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon; 2,3-Butandion, 2,4-Pentandion, 2,5-Hexandion und 5-Methylvinylketon.

Darüber hinaus können Carbonsäuren und Derivate davon, vorzugsweise solche mit 1-20 Atomen umgesetzt werden. Insbesonders sind die folgenden zu nennen: Carbonsäuren, wie z.B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Mitrobenzoesäure, Salicylsäure, p-Hydroxybenzoesäure, Anthranilsäure, p-Aminobenzoesäure, Oxalsäure, Maleinsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;

Carbonsäureester, wie z.B. die C₁-C₁₀-Alkylester der oben genannten Carbonsäuren, insbesondere Methylformiat, Essigester, Buttersäurebutylester, Terephthalsäuredimethylester, Adipinsäuredimethylester, Maleinsäuredimethylester, (Meth)acrylsäuremethylester, Butyrolacton, Caprolacton und Polycarbonsäureester, wie z.B. Polyacryl- und Polymethacrylsäureester und deren Copolymere und Polyester, wie z.B. Polymethylmethacrylat, Terephthalsäureester und andere technische Kunststoffe;

Fette;

Carbonsäureanhydride, wie z.B. die Anhydride der oben genannten Carbonsäuren, insbesondere Essigsäureanhydrid, Propionsäureanhydrid, Benzoesäureanhydrid und Maleinsäureanhydrid,

Carbonsäureamide, wie z.B. Formamid, Acetamid, Propionamid, Stearamid, Terephthalsäureamid.

Ferner können auch Hydroxycarbonsäuren, wie z.B. Milch-, Äpfel-, Wein- oder Zitronensäure, oder Aminosäuren, wie z.B. Glycin, Alanin, Prolin und Arginin, und Peptide umgesetzt werden.

Besonders bevorzugt wird das erfindungsgemäße Verfahren zur Hydrierung von Estern, Anhydriden, Aldehyden und Hydroxyaldehyden eingesetzt.

Die zu hydrierende Carbonylverbindung kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF, NMP, sowie Ether, wie z.B. Dimethyl-, Diethylether, MTBE, besonders bevorzugt ist Wasser.

Die Hydrierung von unter Reaktionsbedingungen flüssigen Edukten führt man sowohl in Sumpf- als auch in Rieselfahrweise, wobei jeweils bevorzugt in Kreislauffahrweise gearbeitet wird, im allgemeinen bei einer Temperatur von 50 bis 250°C, bevorzugt bei 70 bis 200°C, besonders bevorzugt bei 100 bis 140°C und einem Druck von 15 bis 250 bar, bevorzugt 20 bis 200 bar, besonders bevorzugt 25 bis 100 bar durch. Gasphasenhydrierungen werden üblicherweise bei Temperaturen von 120 bis 350°C, bevorzugt 180 bis 300°C und einem Druck von 1 bis 100 bar, bevorzugt 1 bis 60 bar und insbesondere von 2 bis 20 bar durchgeführt.

Die erfindungsgemäß verwendeten Katalysatoren besitzen im allgemeinen eine ausreichende Standzeit. Für den Fall, dass die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, die Katalysatoren mit geeigneten Lösungsmitteln zu spülen.

Mit dem Hydrierverfahren unter Verwendung des erfindungsgemäß werden hohe Umsätze und Selektivitäten erzielt, und die Katalysatoren weisen eine hohe chemische Stabilität in Gegenwart des Reaktionsgemisches auf. Die erfindungsgemäß hergestellten Katalysatoren weisen sowohl im oxidischen als auch im reduzierten Zustand eine deutlich höhere mechanische Stabilität, wodurch sich das erfindungsgemäße Verfahren durch eine besondere Wirtschaftlichkeit auszeichnet.

Die Erfindung wird in den nachstehenden Beispielen näher erläutert.

### Messung BET-Oberfläche

Die Bestimmung der BET-Oberflächen wurde durch Adsorption von N2 entsprechend DIN 66131 vorgenommen.

### Messung der Schneidhärte

Die Messung der Schneidhärte wurde wie folgt durchgeführt:
25 zufällig ausgewählte, optisch rissfreie Extrudate werden nacheinander durch eine Schneide von 0,3 mm Stärke mit zunehmender Kraft belastet, bis das jeweilige Extrudat durchtrennt ist. Die dazu benötigte Kraft ist die Schneidhärte N.

### Beispiel 1

### Herstellung einer erfindungsgemäßen Aktivmasse

In einem beheizbaren und mit Rührwerk ausgestatteten Fälltopf werden 1,5 I Wasser vorgelegt und auf 80°C erwärmt. In dieses Fällgefäß werden im Verlauf einer Stunde eine Metallsalzlösung bestehend aus 877 g Cu(NO₃)₂*2,5H₂O und 551 g Al(NO₃)₃*9H₂O in 2000 ml Wasser und gleichzeitig Sodalösung (20 gew.-%ig) unter Rühren zudosiert. Die Sodadosierung wird so gewählt, dass sich im Fällgefäß ein pH-Wert von 6 einstellt. Nach vollständiger Zugabe der Metallsalzlösung wird weiter Sodalösung zudosiert, bis im Fällgefäß ein pH-Wert von 8 erreicht ist und weitere 15 min bei diesem pH-Wert gerührt. Der Gesamtverbrauch an Sodalösung liegt bei 3,7 kg. Die gebildete Suspension wird abfiltriert und mit Wasser gewaschen, bis das ablaufende Waschwasser kein Nitrat (< 25 ppm) mehr enthält. Das Produkt wird bei ca. 120°C getrocknet. Die so hergestellte Aktivmasse enthält gerechnet als Oxide rund 80 Gew.-% CuO und 20 Gew.-% Al₂O₃.

### Herstellung der Extrudate

133 g Böhmit (Typ Versal 250, Fa.Sasol) wird in einem Mix-Muller mit Ameisensäure (30 gew.-%ig) angeätzt, mit der getrockneten Aktivmasse vermischt und nach Zugabe von 233 ml Wasser intensiv vermischt. Anschließend wird die Knetmasse zu Strängen mit einem Durchmesser von 2 mm und einer mittleren Länge von 8 mm extrudiert. Die Stränge werden anschließend bei ca. 120°C getrocknet und bei 600°C calciniert. Die Katalysatorextrudate enthalten gerechnet als Oxid rund 65 Gew.-% CuO und 35 Gew.-% Al₂O₃. Ca. 54% des oxidischen Trägermaterials Al₂O₃ im Katalysator entstammen aus dem Binder.

Die wichtigsten Katalysatoreigenschaften sind in Tabelle 2 zusammengefasst.

Hydrierung von Maleinsäureanhydrid (MSA) zu Mischungen aus Tetrahydrofuran (THF) und gamma-Butyrolacton (GBL) am erfindungsgemäßen Katalysator

Vor Reaktionsbeginn wird der Katalysator einer Wasserstoffbehandlung unterzogen. Hierzu wird der Reaktor auf 180°C temperiert und der Katalysator während der in Tabelle 1 angegebenen Zeit mit dem jeweils angegebenen Gemisch aus Wasserstoff und Stickstoff bei Atmosphärendruck reduziert.

**Tabelle 1**

| Zeit | Wasserstoff | Stickstoff |
|---|---|---|
| (Minuten) | (NI/h) | (NI/h) |
| 120 | 50 | 950 |
| 30 | 100 | 900 |
| 30 | 500 | 500 |

Anschließend wird der Katalysator 1 h bei 280°C mit 200 NI/h Wasserstoff behandelt.

Für die Durchführung der Hydrierung wird aufgeschmolzene MSA im Gegenstrom auf einen bei 245°C betriebenen und mit Wasserstoff durchströmten Verdampfer gepumpt. Der Gasstrom aus Wasserstoff und MSA gelangt in den mit einem Gemisch aus 70 ml Katalysator und 70 ml Glasringen gefüllten, temperierten Reaktor (Durchmesser 27 mm); der gasförmige Reaktoraustrag wird gaschromatographisch quantitativ analysiert. Die Betriebsparameter und Versuchsergebnisse sind Tabelle 3 zu entnehmen.

### Beispiel 2

### Herstellung einer erfindungsgemäßen Aktivmasse

In einem beheizbaren und mit Rührwerk ausgestatteten Fälltopf werden 1,5 I Wasser vorgelegt und auf 80°C erwärmt. In dieses Fällgefäß werden im Verlauf einer Stunde eine Metallsalzlösung bestehend aus 877g Cu(NO₃)₂*2,5H₂O und 1410g Al(NO₃)₃*9H₂O in 2000 ml Wasser und gleichzeitig Sodalösung (20 gew.-%ig) unter Rühren zudosiert. Die Sodadosierung wird so gewählt, dass sich im Fällgefäß ein pH-Wert von 6 einstellt. Nach vollständiger Zugabe der Metallsalzlösung wird weiter Sodalösung zudosiert, bis im Fällgefäß ein pH-Wert von 8 erreicht ist und weitere 15 min bei diesem pH-Wert gerührt. Der Gesamtverbrauch an Sodalösung liegt bei 4,4 kg. Die gebildete Suspension wird abfiltriert und mit Wasser gewaschen, bis das ablaufende Waschwasser kein Nitrat (< 25 ppm) mehr enthält. Das Produkt wird bei ca. 120°C getrocknet. Die so hergestellte Aktivmasse enthält gerechnet als Oxide rund 61 Gew.-% CuO und 39 Gew.-% Al₂O₃.

### Herstellung der Extrudate

160 g Böhmit (Typ Versal 250, Fa.Sasol) wird in einem Mix-Muller mit Ameisensäure (30 gew.%ig) angeätzt, mit der getrockneten Aktivmasse vermischt und nach Zugabe von 327 ml Wasser intensiv vermischt. Anschließend wird die Knetmasse zu Strängen mit einem Durchmesser von 2 mm und einer mittleren Länge von 8 mm extrudiert. Die Stränge werden anschließend bei ca. 120°C getrocknet und bei 600°C calciniert. Die Katalysatorextrudate enthalten gerechnet als Oxid rund 50 Gew.-% CuO und
50 Gew.-% Al₂O₃. Ca. 36% des oxidischen Trägermaterials Al₂O₃ im Katalysator entstammen aus dem Binder.

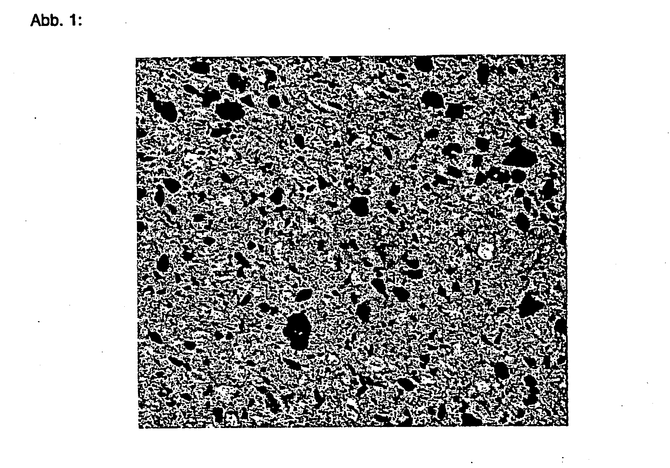

Die wichtigsten Katalysatoreigenschaften sind in der Tabelle 2 zusammengefasst. Die Ergebnisse eines Hydrierexperiments entsprechend Beispiel 1 zeigt Tabelle 3, eine elektronenmikroskopische Abbildung des Katalysatorstrangs nach Reduktion und Rexidation zeigt Abb. 1.

### Vergleichsbeispiel 1

### Herstellung der Extrudate

88,7g Böhmit (Versal 250, Fa. Sasol) werden in einem Laborkneter mit Ameisensäure (30 gew.%ig) angeätzt, mit 83,4 g basischen Kupfercarbonat (Malachit; Cu-CO3*Cu(OH)2; Fa. Aldrich) vermischt und nach Zugabe von 80 ml Wasser intensiv vermischt. Anschließend wird die Knetmasse zu Strängen mit einem Durchmesser von 2 mm und einer mittleren Länge von 8 mm verstrangt. Die Stränge werden anschließend bei ca. 120°C getrocknet und bei 600°C calciniert. Die Extrudate enthalten 50 Gew.-% CuO und 50 Gew.-% Al₂O₃ Die Aktivmasse enthält bei diesem Beispiel keinen Trägeranteil; der Träger stammt damit ausschließlich aus dem eingesetzten Binder.

Die wichtigsten Katalysatoreigenschaften sind in der Tabelle 2 zusammengefasst. Die Ergebnisse eines Hydrierexperimenis unter Verwendung des nicht-erfindungsgemäßen Katalysators V1 entsprechend Beispiel 1 zeigt Tabelle 3.

**Tabelle 2: Zusammenstellung der Katalysatordaten**

| Beispiel | Litergewicht [g/l] | Schneidhärte [N] | Duchmesser [mm] | BET [m2/g] | Porenvolumen [cm3/g] | |
|---|---|---|---|---|---|---|
| | | | | | gesamt | Für D=10-100nm |
| 1 | 906 | 25 | 1,7 | 104 | 0,31 | 0,24 |
| 2 | 790 | 20 | 1,7 | 121 | 0,48 | 0,36 |
| V1 | 620 | 11 | 1,8 | 155 | 0,59 | 0,13 |

**Tabelle 3: Zusammenstellung der Hydrierergebnisse**

| Bsp. | p [bar] | T Hotspot [°C] | Belastung [kg MSA/ I Kalt * h] | GHSV [1/h] | C (MSA) im Feed [Vol-%] | U (MSA) [mol-%] | S (BSA) [mol-%] | S (THF) [mol-%] | S (GBL) [mol%] | S (BuOH) [mol%] |
|---|---|---|---|---|---|---|---|---|---|---|
| 1 | 10 | 250 | 0,15 | 2800 | 1,25 | 100 | 0,7 | 51,6 | 45,8 | 0,3 |
| 2 | 10 | 250 | 0,17 | 3200 | 1,25 | 100 | 1,5 | 49,3 | 47,6 | 0,1 |
| V 1 | 10 | 250 | 0,03 | 700 | 1,15 | 100 | 60,6 | 1,0 | 32,4 | 0 |

| | | | | | | | | | | |
|---|---|---|---|---|---|---|---|---|---|---|
| GHSV Gas hourly Space Velocity = Volumenstrom des Reaktionsgases bei Normbedingungen bezogen auf das Katalysatorvolumen U Umsatz S Selektivität | | | | | | | | | | |

Wie deutlich zu erkennen ist, zeigen die erfindungsgemäßen Katalysatoren hervorragende Selektivitäten zu den Wertprodukten THF und GBL. Der Vergleichskatalysator liefert trotz drastisch geringerer Belastung immer noch hohe Mengen an Bernsteinsäureanhydrid (BSA), ist also in seiner Aktivität deutlich reduziert.

Abbildung 1 zeigt eine rasterelektronenmikroskopische Aufnahme eines Katalysatorstrangs gemäß Beispiel 2 (Aufnahme der Rückstreuelektronen eines Schliffs; Ausschnitt der Abb. ca. 170x170 µm²). In den dunklen Bereichen wird mittels EPMA-Analyse praktisch ausschließlich Aluminium detektiert (bei dieser RE-Darstellung erscheinen Bereiche höherer Dichte, z.B. bedingt durch einen hohen Kupferanteil, heller); in den anderen Bereichen findet sich sowohl Aluminium als auch Kupfer. Eine Flächenauswertung der dunklen Partikel ergibt einen Anteil an partikulärem Aluminiumoxid in dieser Probe von ca. 18 %; unter der Annahme, dass der Schliff für die Probe repräsentativ ist, entspricht dies auch ca. 18 Vol.-% partikulärem Al₂O₃ im gesamten Katalysatorstrang.

## Patentansprüche

1. Katalysatorformkörper von makroskopisch einheitlichem Strukturaufbau enthaltend 5 bis 85 Gew.-% Kupferoxid und ein oxidisches Trägermaterial, der in der Aktivmasse und als Binder das gleiche oxidische Trägermaterial aufweist, **dadurch gekennzeichnet, dass**
a) der Formkörper ein Porenvolumen > 0,15 ml/g im Bereich der Porendurchmesser von 10nm bis 100 nm zeigt und
b) das oxidische Trägermaterial im Formkörper sowohl feindispers verteilt vorhanden ist, als auch zu einem Volumenanteil von 1 bis 95 Vol.-% des Formkörpers in partikulärer Form, vorliegt.

2. Katalysator nach Anspruch 1, **dadurch gekennzeichnet, dass** als oxidisches Trägermaterial Aluminiumoxid, Titanoxid, Zirkonoxid, Siliciumoxid, Manganoxid oder deren Mischungen verwendet werden.

3. Katalysator nach Anspruch 1 oder 2, **dadurch gekennzeichnet, dass** das oxidische Trägermaterial Al₂O₃ ist.

4. Katalysator nach Anspruch 3, **dadurch gekennzeichnet, dass** das Al₂O₃ vorwiegend als röntgenamorphes Material vorliegt.

5. Katalysator nach einem der Ansprüche 1 bis 4, **dadurch gekennzeichnet, dass** es sich um ein Extrudat handelt.

6. Verfahren zur Herstellung eines Katalysators nach einem der Ansprüche 1 bis 5, **dadurch gekennzeichnet, dass** eine Aktivkomponente enthaltend 10 bis 98 Gew.-% Kupferoxid und ein oxidisches Trägermaterial mit einem Binder, enthaltend das gleiche Trägermaterial oder einen Precursor davon vermischt und zu Formkörpern verformt wird.

7. Verfahren gemäß Anspruch 6, **dadurch gekennzeichnet, dass** der Anteil an oxidischem Trägermaterial im Katalysator zu 10 bis 98 Gew.-% aus dem eingesetzten Binder entstammt.

8. Verwendung eines Katalysators nach einem der Ansprüche 1 bis 5 zur Hydrierung von Carbonylverbindungen.

9. Verwendung eines Katalysators nach einem der Ansprüche 1 bis 5 zur Gasphasenhydrierung von Maleinsäureanhydrid.

## Claims

1. A shaped catalyst body having a macroscopically uniform structure and comprising from 5 to 85% by weight of copper oxide and an oxidic support material, which has the same oxidic support material in the active composition and as binder, wherein
a) the shaped body has a pore volume of > 0.15 ml/g in the pore diameter range from 10 nm to 100 nm and
b) the oxidic support material in the shaped body is present both in finely disperse form and also to a proportion by volume of from 1 to 95% by volume of the shaped body in particulate form.

2. The catalyst according to claim 1, wherein the oxidic support material used is aluminum oxide, titanium oxide, zirconium oxide, silicon oxide, manganese oxide or a mixture thereof.

3. The catalyst according to claim 1 or 2, wherein the oxidic support material is Al₂O₃.

4. The catalyst according to claim 3, wherein the Al₂O₃ is predominantly present as X-ray-amorphous material.

5. The catalyst according to any of claims 1 to 4 which is an extrudate.

6. A process for producing a catalyst according to any of claims 1 to 5, wherein an active component comprising from 10 to 98% by weight of copper oxide and an oxidic support material is mixed with a binder comprising the same support material or a precursor thereof and shaped to form shaped bodies.

7. The process according to claim 6, wherein from 10 to 98% by weight of the oxidic support material in the catalyst comes from the binder used.

8. The use of a catalyst according to any of claims 1 to 5 for the hydrogenation of carbonyl compounds.

9. The use of a catalyst according to any of claims 1 to 5 for the gas-phase hydrogenation of maleic anhydride.

## Revendications

1. Corps moulé catalytique d'une structure macroscopiquement uniforme, contenant 5 à 85 % en poids d'oxyde de cuivre et un matériau support oxydique, qui comporte le même matériau support oxydique dans la masse active et en tant que liant, **caractérisé en ce que**
a) le corps moulé présente un volume poreux > 0,15 ml/g dans la plage des diamètres de pore de 10 nm à 100 nm et
b) le matériau support oxydique est présent dans le corps moulé sous forme finement dispersée et également, en une proportion volumique de 1 à 95 % en volume du corps moulé, sous forme particulaire.

2. Catalyseur selon la revendication 1, **caractérisé en ce que** de l'oxyde d'aluminium, de l'oxyde de titane, de l'oxyde de zirconium, de l'oxyde de silicium, de l'oxyde de manganèse ou leurs mélanges sont utilisés en tant que matériau support oxydique.

3. Catalyseur selon la revendication 1 ou 2, **caractérisé en ce que** le matériau support oxydique est Al₂O₃.

4. Catalyseur selon la revendication 3, **caractérisé en ce que** l'Al₂O₃ se présente principalement sous la forme d'un matériau amorphe par rayons X.

5. Catalyseur selon l'une quelconque des revendications 1 à 4, **caractérisé en ce qu'**il s'agit d'un extrudat.

6. Procédé de fabrication d'un catalyseur selon l'une quelconque des revendications 1 à 5, **caractérisé en ce qu'**un composant actif contenant 10 à 98 % en poids d'oxyde de cuivre et un matériau support oxydique sont mélangés avec un liant, contenant le même matériau support ou un précurseur de celui-ci, et mis sous la forme de corps moulés.

7. Procédé selon la revendication 6, **caractérisé en ce que** la proportion de matériau support oxydique dans le catalyseur est issue à 10 à 98 % en poids du liant utilisé.

8. Utilisation d'un catalyseur selon l'une quelconque des revendications 1 à 5 pour l'hydrogénation de composés de carbonyle.

9. Utilisation d'un catalyseur selon l'une quelconque des revendications 1 à 5 pour l'hydrogénation en phase gazeuse d'anhydride de l'acide maléique.
